# EUROPEAN PATENT SPECIFICATION

(11) **EP 4 312 900 B1**
(45) Date of publication and mention of the grant of the patent: **13.08.2025**
(21) Application number: 22718692.1
(22) Date of filing: 28.03.2022
(51) Int. Cl.: A61F 5/00

(54) **GASTRO-INTESTINAL TUBE AND ANCHORING THEREFOR**
GASTROINTESTINALTUBUS UND VERANKERUNG DAFÜR
TUBE GASTRO-INTESTINAL ET ANCRAGE ASSOCIÉ

(30) Priority: 26.03.2021 EP 21165408; 09.07.2021 EP 21315126
(43) Date of publication of application: 07.02.2024
(73) Proprietor: BariaTek Medical, 75013 Paris (FR)
(72) Inventor: BASTID, Christophe, 13009 Marseille (FR); GARD, Marco, 10031 Borgomasino (IT); MANOS, Thierry, 13007 Marseille (FR); SEJOR, Eric, 06000 Nice (FR); BIADILLAH, Youssef, 78600 Maisons-Laffitte (FR)
(74) Representative: Hepp Wenger Ryffel AG
(86) International application number: PCT/EP2022/058125
(87) International publication number: WO 2022/200630

(56) References cited:
- WO-A1-2013/023675
- US-A1- 2014 378 884

## Description

The present invention relates to the field of tubes for insertion into the gastro-intestinal tract, and to anchors therefor. In some non-limiting aspects, the tubes are bypass tubes for bypassing portions of the bowel.

Various surgical techniques, and implants, have been proposed for treating obesity and diabetes. Surgical techniques include creation of gastric pockets and gastric bypasses of the stomach, duodenum and part of the jejunum. Bypass tubes or liners have been proposed for insertion into the gastro-intestinal tract, to bypass the duodenum and optionally part of the jejunum.

Technical challenges remain for many of these techniques. For example, the endoscopic placement and anchoring of bypass tubes remains challenging. One technique proposed is to anchor the tube in the vicinity of the pylorus. However, the stomach and intestine are subject to significant motion in normal bodily function. Muscular contractions of the stomach, including at the pyloric antrum, complicate maintaining the tube in position. The muscular contractions can be extreme in the case of, for example, the patient vomiting. Dislodgement either towards the duodenum, or into the stomach, can necessitate medical intervention to correct the position or to retrieve the bypass tube. Many existing proposals are compromised by the apparently conflicting need for secure anchoring, yet with atraumatic engagement with the body tissue to avoid the device causing tissue irritation.

It would be desirable to address and/or mitigate one or more of the above issues.

Aspects of the invention are defined in the claims.

Non-limiting embodiments of the invention are now described, by way of example only, with reference to the accompanying drawings, in which:
Fig. 1 is a schematic side view of a first example of a gastric anchor for a duodenal tube.
Fig. 2 is a schematic section illustrating the first example in an implanted condition.
Fig. 3 is a schematic side view if a second example of a gastric anchor similar to Fig. 1.
Fig. 4 is a schematic section illustrating in an implanted condition a further example similar to the first and second examples.
Fig. 5 is a schematic section illustrating in an implanted condition a further example having at least one balloon.
Fig. 6 is a schematic section illustrating in an implanted condition a further example having at least one balloon.
Fig. 7 is a schematic section illustrating in an implanted condition a further example having multiple balloons.
Fig. 8 is a schematic section illustrating a further example of an anchor for anchoring at the pylorus.
Fig. 9 is a schematic section illustrating a detail of Fig. 8.
Fig. 10 is a schematic section illustrating a further example of an anchor similar to that of Figs. 8 and 9.
Fig. 11 is a schematic side view of a further example of tube attachable by sutures and/or staples.
Figs. 12a-d are schematic sections illustrating steps for attaching a tube to a stomach wall with pledgets.
Figs 13a and 13b are schematic perspective views of an exterior profile of a tube in a further example.
Fig. 14 is a schematic view of a tube placed at a gastro-jejunal anastomosis.
Fig. 15 is a schematic section of the tube of Fig. 14 in isolation.
Fig. 16 is a schematic section illustrating formation of pleats to reduce stomach volume.
Fig. 17 is a schematic section illustrating a detail of Fig. 16.
Fig. 18 is a schematic section illustrating the stomach pleats in combination with other treatment devices for diverting flow of stomach contents.
Fig. 19 is a schematic side view of one example of tissue-penetrating fixing.
Fig. 20 is a schematic side view of a second example of tissue-penetrating fixing.
Fig. 21 is a schematic side view of a needle carrying the tissue-penetrating fixing for deployment.
Fig. 22 is a schematic section showing the needle of Fig. 21.
Fig. 23 is a schematic side section of a working end of a tool for attaching a duodenal tube to a pyloric sphincter using the tissue-penetrating fixings of Figs. 19 or 20.
Fig. 24 is a schematic perspective view of the working end of the tool of Fig. 23.
Figs. 25-32 are schematic side views illustrating a sequence for placing an attaching a bypass tube within the duodenum using the tool of Figs. 23 and 24.
Fig. 33 is a schematic side view illustrating a second example using two attachment rings.

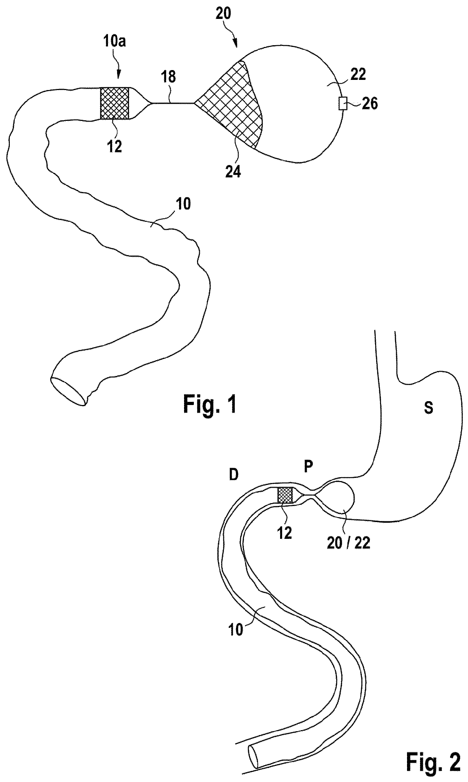

In the following description, the same reference numerals are used to denote similar or equivalent features, whether or not described in detail. The description of one embodiment can thus be combined with another embodiment. Where a tube is described, the tube may optionally be a bypass tube or bypass conduit for bypassing a portion of the bowel to reduce nutrient uptake. Referring to Figs. 1 and 2, a tube 10 is illustrated for introduction into at least the duodenum D of a patient, for the treatment of diabetes or obesity. The tube 10 optionally may extend to the jejunum. A proximal end 10a of the tube may be disposed in the duodenum D close to the pylorus P as shown in Fig. 2, or it may extend at least partly through the pylorus P. The tube 10 comprises a cuff or stent 12 for resisting displacement from the implanted position towards the stomach. The tube 10 may optionally comprise reinforcement (not shown in Figs 1 and 2, but reference 14 in later figures) for preventing twisting of the tube 10 at the pylorus that could create a blockage.

An anchor 20 for the tube 10 comprises a gastric bulb 22 for placement with the stomach S. The gastric bulb 22 has a collapsed state suitable for introduction into the stomach, for example, through the patient's mouth, and an operative state in which the bulb 22 bulges to define an anchor that resists displacement through the pylorus P.

The anchor 20 is coupled, or couplable, to the tube 10 by any suitable coupling, for example, by one or struts or one or more flexible tethers 18. Alternatively, the anchor 20 is mounted directly on the tube 10.

In the illustrated examples, the bulb 22 is configured such that, in the expanded condition a pressure within the bulb is (i) not greater than atmospheric pressure, and/or (ii) not greater than surrounding pressure within the stomach. The bulb 22 is not permanently substantially pressurized compared to the surrounding ambient pressure within the stomach. This contrasts with a type of balloon or bulb that is inflated, and distends from within by virtue of inflation pressure.

By virtue of, for example, absence of a positive pressure within the bulb 22, the bulb 22 is able to partly collapse in response to stomach contractions. This enables the bulb 22 to exhibit, at least partly, compliance with the stomach contractions, and to absorb the contractions without pulling overly on the tube 10, which might otherwise dislodge the tube 10 into the stomach. The bulb 22 can partly compress to absorb or accommodate extreme transient stomach contractions, for example, should the patient vomit. At the same time, the bulb 22 is biased to its operative condition in which is serves to prevent displacement in the distal direction, as the bulb 22 will resist passage towards and through the pylorus.

In the illustrated examples, the bulb 22 is self-expandable from the collapsed condition to the operative condition. The gastric bulb 22 can comprise a self-expandable structure 24 (depicted schematically in part in Fig. 2), optionally a frame structure (e.g. with ribs and/or struts), and/or a mesh structure.

In one version, the gastric bulb 22 may be open to its interior and/or have communication apertures allowing chyme to pass into the interior of the bulb 22. For example, the bulb 22 may comprise an open frame 24.

Alternatively, the bulb 22 may comprise a fluid-tight chamber. The chamber may have an inlet port 26 for allowing fluid to be drawn into the chamber as the gastric bulb 22 expands to its operative condition under the influence of the self-expanding property, for example, provided by self-expanding frame 24. A delivery device may include an inflow conduit (e.g. vent channel) removably coupled to the inlet port 26 to allow pressure equalization and/or inflation by admission of fluid. The fluid may, for example, be a liquid (e.g. saline) or a gas (e.g. air). After pressure equalization and/or inflation is complete, the inflow conduit can be disconnected from the inlet port 26, thus sealing the chamber. The small quantity of fluid in the chamber can provide a compliant cushion that supplements the self-expanding frame 24, providing an atraumatic anchor with compliant characteristics. Optionally, the fluid-tight chamber may include a central passage (not shown) for allowing chyme to pass towards the pylorus and the entrance to the tube 10.

Where both a self-expanding structure and a fluid-tight chamber are provided, the self-expanding structure and the fluid-tight chamber may be nest one at least partly (optionally substantially entirely) within the other. For example, the fluid-tight chamber may be nested at least partly (optionally substantially entirely) with the self-expanding structure. A surface (e.g. outer surface) of the fluid-tight chamber may be in surface-to-surface contact (e.g. laminated) with a surface (e.g. inner surface) of the self-expanding structure.

With or without self-expansion, the bulb may comprise a fluid-tight chamber without a distinct self-expanding structure. The fluid-tight chamber may have an inlet port for admitting fluid into the chamber. A further alternative for self-expansion is for the fluid-tight chamber to be made of resilient material that self-expands to a deployed configuration, for example, towards a moulded configuration of the fluid-tight chamber.

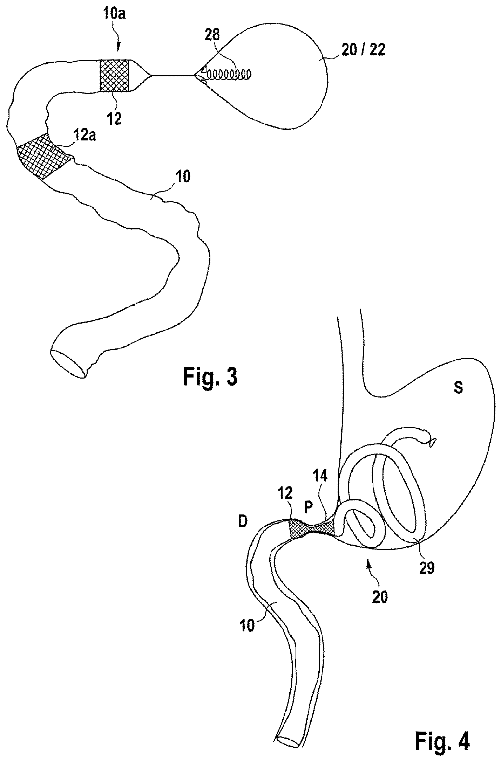

Fig. 3 illustrates a second example similar to the first example of Figs. 1 and 2. The main difference in Fig. 3 is the addition of an adjustment mechanism 28 for enabling the distance between the bulb 22 and the proximal end 10a of the tube 10 to be set prior to introduction into the stomach and/or in situ during or after placement in the stomach. The adjustment mechanism 28 may, for example, comprise a screw thread and a rotatable nut. The adjustment mechanism 28 may be adjusted may manual rotation using a suitable tool, or by magnetic rotation using a magnetic tool.

Referring to Fig. 4, an alternative example of anchor 20 is illustrated comprising at least one resilient element 29, expandable from a collapsed condition for introduction into the stomach, and an expanded operative condition (Fig. 4) in which the resilient element 30 has an at least partly helix shape.

The helix shape may diverge in a radial direction progressively away from the proximal end of the tube. The helix shape may include open space between adjacent turns or windings of the helix.

Such a configuration may be advantageous to be easily collapsed for introduction, while also expanding to a relatively large size in its operative condition. The helix shape can provide atraumatic compliant engagement able to absorb and accommodate stomach contractions, even strong transient contractions, without pulling overly on the tube 10. At the same time, the helix shape reliably anchors the tube 10 to prevent displacement through the pylorus. The anchor 20 can be collapsed by pulling on the free end of the helix shape using a suitable retrieval tool, allowing the device to be removed when desired.

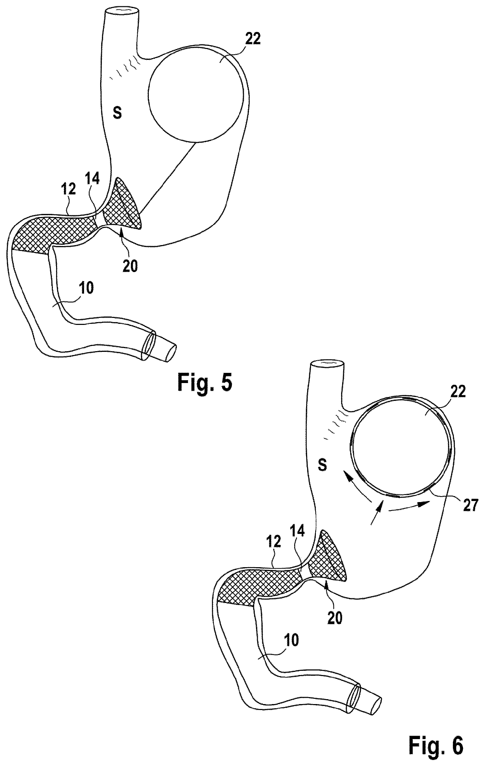

Figs. 5 to 7 illustrate further examples including one or more gastric bulbs or balloons 22 configured to induce a feeling of satiety through contact with the stomach wall. The balloons may or may not function as gastric anchors, depending on the implementation. Where appropriate a gastric anchor 20 is coupled to the proximal end of the tube 10.

The bulbs or balloons 22 may optionally be inflated with fluid (e.g. gas or liquid). A gas, for example air, may desirably cause the balloon to float with respect to stomach contents. In Fig. 5, at least one (optionally more) balloon 22 is tethered to the proximal end of the tube 10, to float upwardly with respect to the stomach contents.

In Fig. 6, at least one (optionally more) balloon 22 has a magnetic property, such as a magnetic coating or carries one or more magnetic elements 27. Magnetic repulsion between the balloon 22 and a same-sense magnetic element (not shown) at the proximal end of the tube 10 urges the balloon 22 away from the tube 10, and towards contact with the stomach wall.

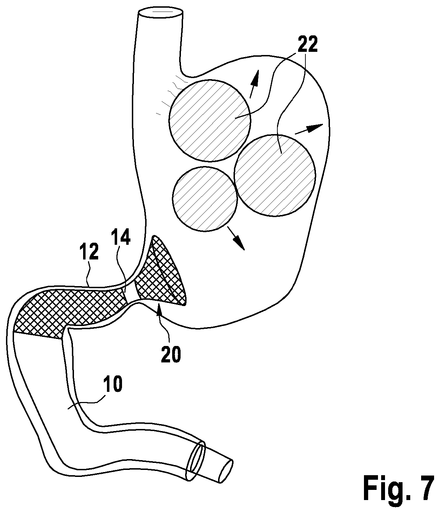

In Fig. 7, multiple balloons 22 occupy significant space within the stomach, and bear against the stomach wall(s). The balloons 22 may be loose and/or captive relative to one another. The balloons 22 may be loose and/or captive with respect to the tube 10.

In the above examples of Figs. 5 to 7, the one or more balloons 22 are sized to permit stomach movements and contractions, and also to permit chyme to pass to the tube 10.

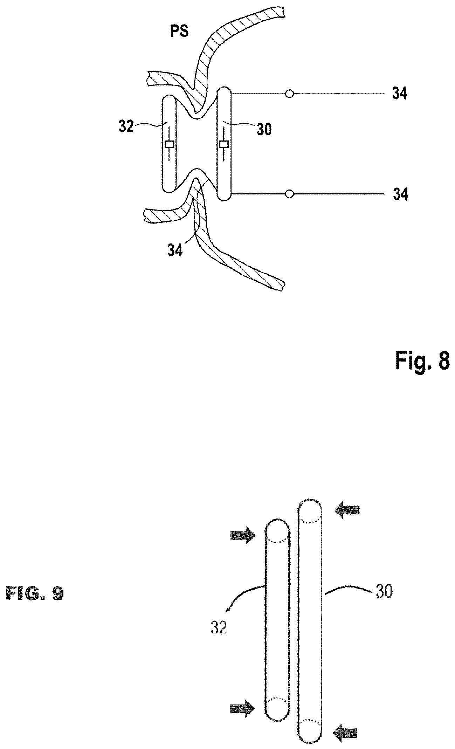

Referring to Figs. 8 to 10, the anchor 20 comprises a first ring 30 for fitting against a stomach-side of a pyloric sphincter PS, and a second ring 32 for fitting against a duodenal-side of the pyloric sphincter PS. The first and second rings 30, 32 are coupled to one another for clamping the pyloric sphincter PS between the rings 30 and 32. As can be seen in the detail of Fig. 9, the first and second rings 30 and 32 have different inner and/or outer diameters from each other. In the illustrated form, one ring (e.g. the first ring 30) has an inner diameter approximately greater than (or at least not substantially smaller than) the outer diameter of the other (e.g. the second ring 32).

Such an anchor 20 firmly, yet atraumatically, attaches to the pyloric sphincter PS, providing good fixation even in the presence of strong stomach contractions. In particular, the offsetting of one ring with respect to another can reduce risk of tissue necrosis that could be caused by pinching between two identical diameter rings with the same clamping force.

The first and/or second rings 30 and 32 may be collapsible to a collapsed condition for introduction to the pylorus. The anchor 20 further comprises one of more connecting elements 34 between the rings 30 and 32, and coupled to the rings 30 and 32 for selectively drawing the rings closer to one another for clamping the pyloric sphincter PS. In the illustrated form, the connecting elements 34 comprise filaments (e.g. sutures). The filaments engage the rings 30 and 32 such that pulling on the filaments draws at least one ring towards the other.

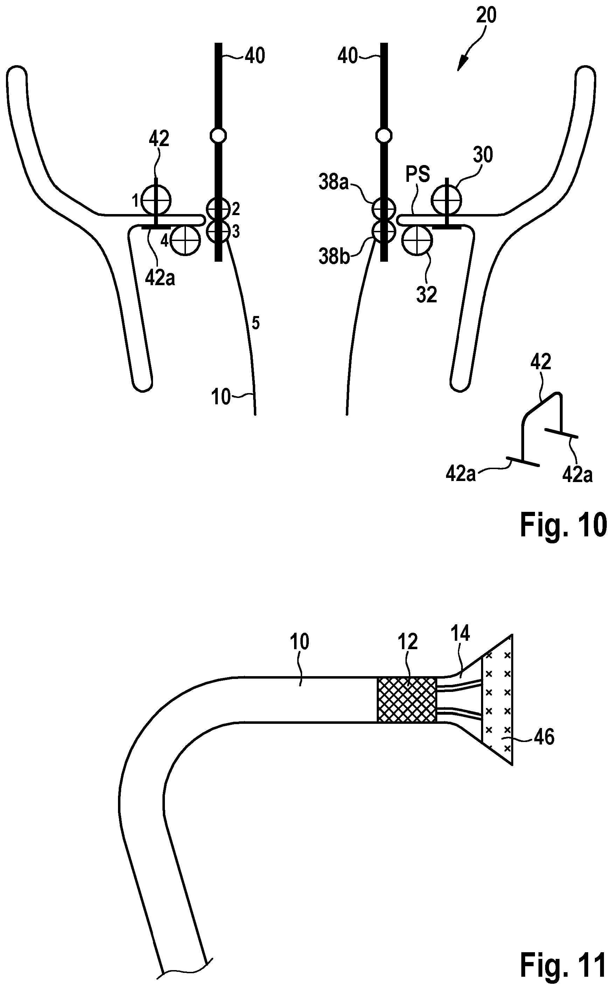

If desired, at least one of the rings 30, 32 may also be directly attached to the pylorus, for example, by one or more tissue-penetrating fixings (similar to that shown in Fig. 10, described below).

The tube (not shown) may be attached to one or more of the first and second rings 30 and 32. The tube may be attached permanently, or via a connector, such as a magnetic connector.

Referring to Fig. 10, in addition to the first and second rings 30 and 32, the anchor 20 further comprises at least one inner ring 38 for corralling an inner peripheral edge of the pyloric sphincter PS. In the illustrated embodiment, first and second inner rings 38a and 38b are used. One of more connecting elements 40 between the inner rings 38a and 38b, and coupled to the rings 38a and 38b selectively draw the rings closer to one another for corralling the inner edge of the pyloric sphincter PS. In the illustrated form, the connecting elements 40 comprise filaments (e.g. sutures). The filaments engage the rings 38a and 38b such that pulling on the filaments draws at least one ring towards the other.

In use, the after placing the second ring 32, the inner rings 38a and 38b may be placed spaced apart from the pyloric sphincter PS. The inner rings 38a and 38b are drawn together to corral the inner edge, followed by placing and/or drawing the first ring 30 to clamp the pylorus between the first and second rings 30 and 32. If desired, one (or both) of the first and second rings 30, 32 may be directly attached to the tissue of the pyloric sphincter, for example, by one or more tissue penetrating fixings 42. In one example, the fixings 42 are staples, for example having a U-shaped form, each limb of the U-shape piercing tissue and held captive by a transverse stop 42a (e.g. defining a T-shape). Alternatively, the fixings 42 may be tag-pins inserted from one side of the tissue, and having a self-expanding transverse stop or wing extending from a central stalk, to hold the fixing captive on the opposite side of the tissue, preventing withdrawal from the first side.

The tube 10 may optionally be attached (e.g. permanently or via an assemblable connection) to one of the rings, for example, one of the inner rings 38a, 38b, as illustrated.

Referring to Fig. 11, a further example of tube 10 is illustrated. The tube 10 is configured for extending from a proximal end 10a at the pylorus into the duodenum and optionally at least partly into the jejunum. The tube 10 may be made of plastics, such as silicone or polyurethane. The proximal end 10a is configured for direct attachment to body tissue by means of sutures or staples 42 that pierce the material of the tube 10. In order to prevent tearing of the plastics material, the proximal end comprises a layer of suture-permeable and/or staple-permeable, reinforcing fabric 46, optionally laminated to and/or incorporated within the plastics material. The fabric 46 may be a woven or non-woven material. The fabric 46 may be of polymer material, such as PET. The natural openings or spaces in the fabric material 46 allow a suture or fixing to pass therethrough without breaking the fiber or filament of the fabric itself. Thus the fabric can prevent tear propagation in the plastics of the tube 10.

The proximal end 10a of the tube 10 may be flared to facilitate placement and attachment of the proximal end 10a.

Optionally, the tube 10 includes a duodenal anchor 12, such as a stent, and/or reinforcement 14 across the pylorus to avoid risk of the tube 10 twisting due to stomach movements.

In use, the tube 10 may be placed into the duodenum and optionally the jejunum. A surgical stapler may be used to staple the proximal end to, for example, tissue forming the pyloric sphincter, in a similar manner to that illustrated in Fig. 10.

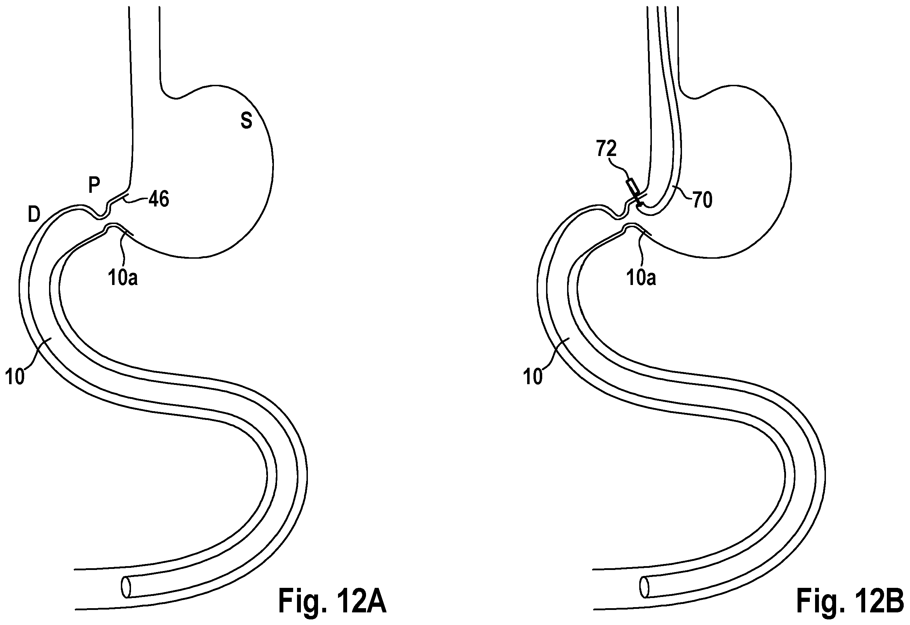

Figs. 12a to 12d illustrate a further technique for fastening the tube 10, such as the tube of Fig. 11. Referring to Fig. 12a, the tube 10 is introduced via the patient's mouth, and placed into at least the duodenum, with the proximal end 10a of the tube 10 proximal of the pylorus. The tube 10 may be held in place temporarily by a mechanical device (not shown).

Referring to Fig. 12b, a tool 70 is guided through the mouth to the pyloric antrum. The tool 70 includes a hypotube needle used to pierce through the region of the tube containing the fabric 46, and through the stomach wall. A pledget 72 having an attached suture is inserted through the fabric 46 and the stomach wall, via the hypotube needle, and deployed outside the stomach wall, leaving the suture trailing through the stomach wall and the fabric 46.

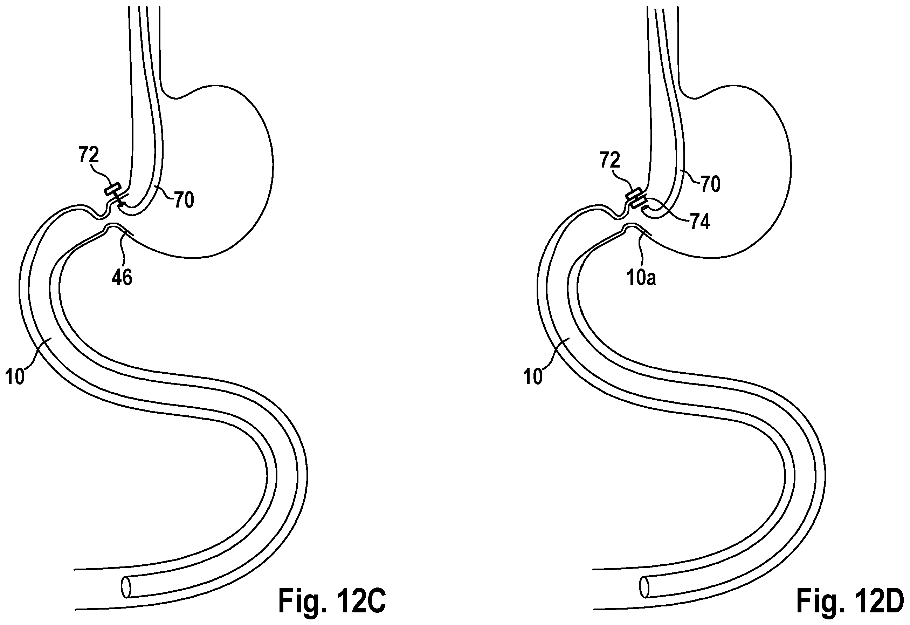

Referring to Figs. 12c and d, the hypotube needle is withdrawn to the interior side of the stomach, and a second pledget 74 is parachuted over the same suture, via the hypotube, and deployed on the interior surface of the stomach wall. The suture is tightened to tightly sandwich the stomach wall and the fabric 46 of the proximal end of the tube, between the two pledgets 72 and 74. The suture is knotted and cut, leaving the pledgets 72 and 74 forming an independent fastening. The same process is repeated at several different points around the periphery of the proximal end of the tube 10 to firmly anchor the tube 10 to the stomach wall. The pledgets can provide a secure attachment of the tube 10 and avoid irritation through rubbing during stomach contractions, because the proximal end of the tube is attached to move intimately with the stomach wall. When it is desired subsequently to remove the tube 10 from the stomach, a tool can be inserted into the stomach to cut the sutures, and release the pledgets 74 on the inside surface of the stomach wall, thereby releasing the tube 10.

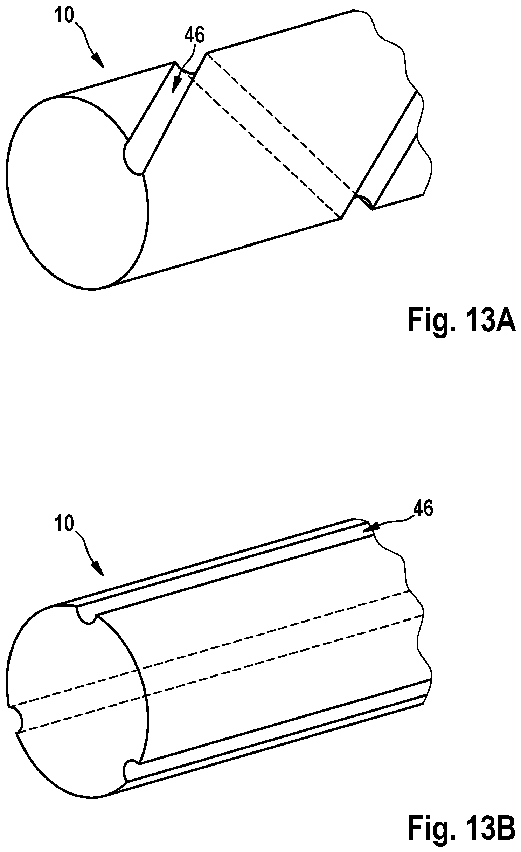

Referring to Figs. 13a and 13b, a tube 10 for insertion into the gastro-intestinal tract, optionally the duodenum, has an outer surface defining at least one channel 46 extending, at least partly, in an axial direction of the tube. In one form, the channel 46 may be substantially axial, for example, in the form of a fluted surface (Fig. 13b). Alternatively, the channel may be helical in shape, in the form of a threaded surface (Fig. 13a). The channel 46 permits bowel juices, for example, pancreatic juices, to flow as in the efferent bowel, along the exterior of the tube 10, and can avoid the juices becoming trapped by contact between the tube 10 and the bowel tissue.

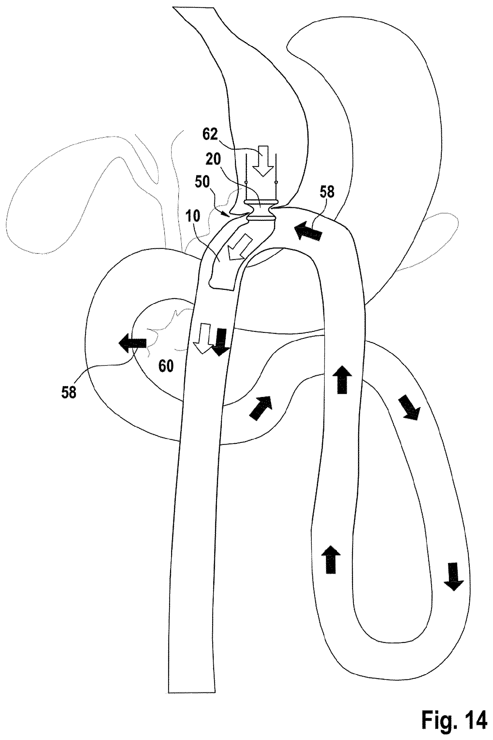

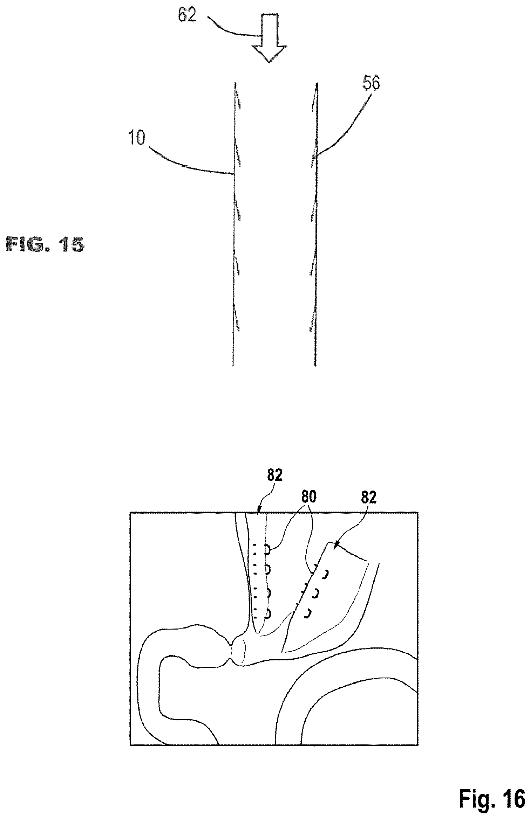

Referring to Figs. 14 and 15, a tube 10 is illustrated for supplementing an anastomosis 50, for example a gastro-jejunal anastomosis. The anastomosis may, for example, be between the bottom of the stomach and the jejunum, or it may be between a cut-down stomach and the jejunum (as illustrated in Fig. 14). The tube 10 may optionally comprise an anchor 20 for anchoring at the anastomosis 50. The anchor may include any of the features described above; optionally the anchor may include the features of any of Figs. 8 to 10.

The tube 10 may function to prevent or reduce reflux of pancreatic juices (illustrated by arrows 58) originating from the pancreas 60 from passing through the anastomosis 50. For example, the tube 10 is placed into the part of the jejunum leading to the large intestine. Stomach contents (arrows 60) passing through the anastomosis are directed in the normal flow direction towards the large intestine. Pancreatic juices arriving (arrows 58) from upstream in the jejunum are directed outside the tube 10 and thus away from the anastomosis 59 and also towards the large intestine, thereby reducing the possibility of reflux of these juices through the anastomosis 50.

The tube 10 may also be configured with a directional flow characteristic, to admit flow of stomach content (e.g. chyme) in a first direction through the tube 10 away from the stomach (arrow 62), and to obstruct flow in an opposite second direction through the tube 10. The one-way characteristic can also block reflux of bowel juices, for example, pancreatic juices in the jejunal loop 54 , through the anastomosis 50 towards the stomach, thereby reducing stomach discomfort for the patient.

The one-way characteristic of the tube may be implemented by at least one, optionally multiple, flaps 56 (Fig. 15) provided on the interior of the tube 10. The flaps may have a flexible and/or loose annular shape, or comprise individual arcuate segments. The flaps 56 are directed towards the outlet. Flow in the first direction (arrow 62) pushes the flaps 56 against the wall of the tube 10 to admit flow. Flow in the reverse direction tends to push the flaps 56 inwardly towards the centre of the channel, to narrow the channel in the tube 10, and hence obstruct flow. Alternatively, the tube 10 may itself be collapsible, like a sock, to perform a one-way valving function. Flow in the first direction opens and distends the tube 10. Flow in the opposite second direction collapses the tube on itself, to obstruct flow.

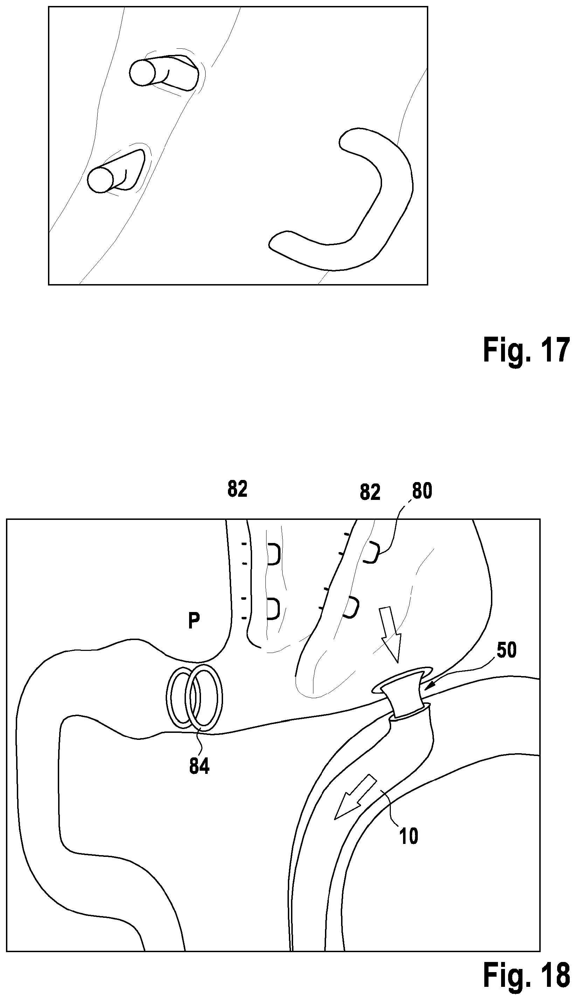

Referring to Figs. 16 and 17, a further illustrated technique for treating diabetes or obesity, involves application of at least one tissue-penetrating fixing 80 to reduce the natural volume of the stomach by creating one or more pleats 82 (or artificial folds) in the stomach wall. In the illustrated example, the fixing 80 is a staple, optionally having a U-shaped profile, with T-shaped limb ends, as described above. The fixing may, for example, be placed by an endoscopic technique or a laparoscopic technique.

The one or more pleats 82 may extend in a direction that is generally (i) from top to bottom of the stomach, and/or (ii) from the oesophagus to the pylorus. Such pleats 82 can form a generally sleeve shape cavity or passage in the stomach.

Referring to Fig. 18, the stomach reduction pleats 82 can also be used in combination with a tube 10, and optionally in combination with an anastomosis 50, for example, a gastro-jejunal anastomosis 50. The pylorus P can be closed or plugged by a closure device 84. The closure device 84 may, for example, comprise rings similar to those described for Figs. 8 to 10 above, for anchoring to the pyloric sphincter.

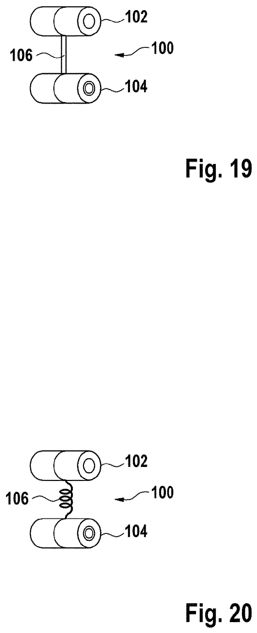

Figs. 19 to 33 illustrate further examples of fixation of a tube 10 to a pylorus P, using tissue-penetrating fixings 100.

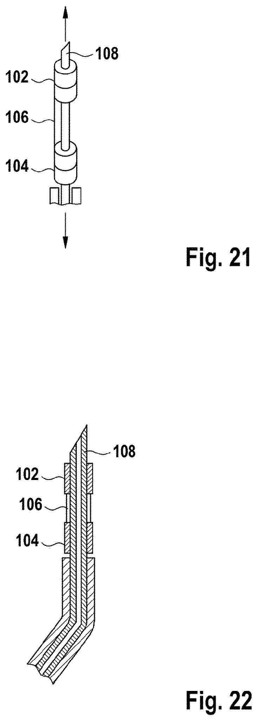

Referring to Figs. 19 and 20, each fixing generally comprises two end pieces in the form of a head 102 and a foot 104 coupled via connecting element 106. The head 102 and foot 104 have a generally atraumatic shape, for example, a rounded rod shape. In the illustrate examples, the head 102 and foot 104 are tubular, enabling the pieces to be carried around a penetration needle 108 (Figs. 21 and 22) for introduction and deployment. The head 102 and foot 104 may be rigid or semi-rigid elements, optionally made of shape memory alloy, for example nitinol.

The connecting element 106 may be rigid or flexible. In the example of Fig. 19, the connecting element 106 is generally linear. In the example of Fig. 20, the connecting element 106 has the form of an elongate spring, for generating a compressive load when the spring is extended.

At least the head 102, and optionally the foot 104 can be brought into a generally non-deployed configuration (Figs. 21 and 22) in which the head (and optionally the foot) extends side by side with the connecting element, for introduction in a streamlined shape. In a deployed configuration (Figs. 19 and 20), the head (and optionally the foot) is moved to project laterally on either side of the connecting member.

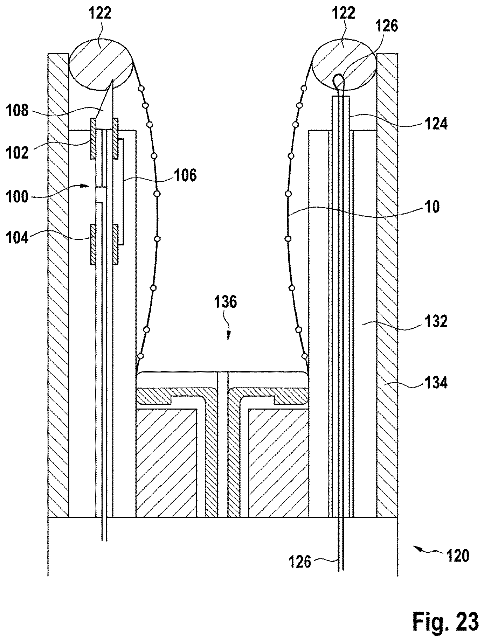

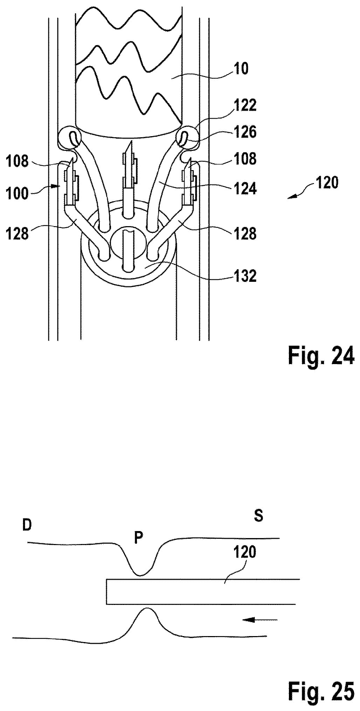

Referring to Figs. 23 and 24, a tool 120 is illustrated for attaching a ring 122 of a duodenal tube 10 to a pylorus P of a patient, using the fixings 100.

The tool 120 comprises a plurality (e.g. three in the illustrated form) of first elongate supports 124 attached to the ring 122 for introducing the ring to the pylorus, and for holding the ring during deployment of the fixings. For example, the first elongate supports 124 may be attached by suture loops 126 to an inner core of the ring 122. The suture loop 126 can pass within the elongate support, and be released from one end when it is desired to separate the elongate support 124 from the ring 122.

The tool 120 further comprises a plurality (e.g. three in the illustrated form) of second elongate supports 128 each having a penetration needle 108 at its free end. A security suture 138 may pass within the the second elongate support, and loop through or around the fixing 100 to ensure that the fixing 100 will not detach prematurely from the needle 108.

The tool 120 further comprises a multi-lumen catheter shaft 132 having lumen for receiving the pluralities of first and second elongate supports 124 and 128. The first and second supports 124 and 128 may be distributed alternately in a circumferential direction, such that each first elongate support 124 is positioned between two adjacent second elongate supports 128, and vice versa. At least the second elongate supports 128 are slidable axially between axially extended and axially retracted positions, for causing the needles 108 to penetrate through tissue, as illustrated later below. The first elongate supports 124 may also be axially slidable if desired.

The tool 120 further comprises a constraining sheath 134 slidable over the catheter shaft 132. The constraining sheath 132 can constrain the first and second elongate supports 124, 128 to a radially compressed configuration (Fig. 23) for introduction through the stomach to the duodenum. Retraction of the constraining sheath 134 allows the first and second elongate supports 124 and 128 to diverge or splay outwardly to a radially enlarged configuration (Fig. 24). The first elongate supports 124 and/or the second elongate supports 126 may be pre-shaped to splay outwardly when the constraining effect of the sheath 134 is removed. The constraining sheath may also serve to restrain the ring 122 in a radially compressed configuration for delivery. In a similar manner to that already described above, the ring may be expandable to its operative size when released from the sheath 134.

The multi-lumen shaft 132 has a hollow interior channel 136 defining an accommodation space for the tube 10. The tool 120 may further comprise a gripper 136 for releasably gripping and controlling the distal end of the tube 10.

Figs. 25 to 32 illustrate a procedure for introducing and attaching a tube 10 through the stomach, using the tool 120. In Fig. 25, the tool 120 housing the tube 10 and the ring 122 in their collapsed configurations, is advanced through the stomach S and through the pylorus P into the duodenum D.

Referring to Fig. 25, the distal end of the tube 10 is advanced out of the sheath 134 by advancing the gripper 136 into the duodenum, over a guidewire G.

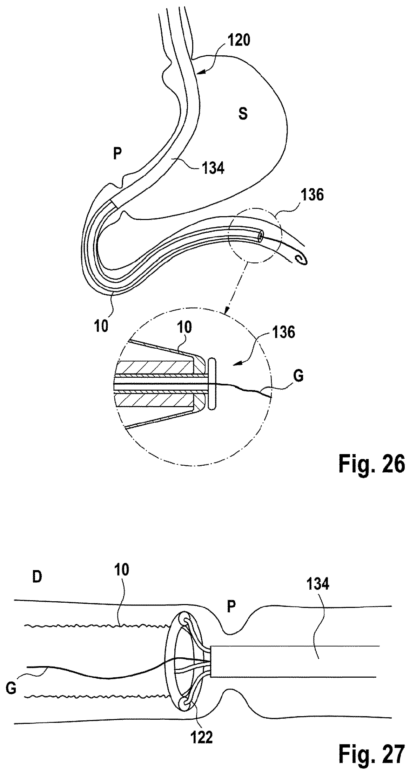

Referring to Fig. 27, the restraining sheath 134 is retracted with respect to the shaft 132, to release the ring 122 attached to the proximal end of the tube 10, and allow the ring 122 to expand towards its functionally operative size.

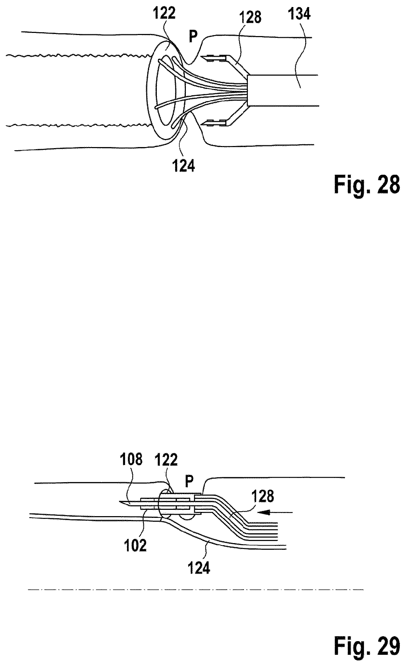

Referring to Fig. 28, pulling slightly on the first elongate supports 124 can seat the ring 122 against the pyloric sphincter tissue on a first (duodenal side). Further retraction of the sheath 134 allows the second elongate supports 128 to expand to present the needles 108 carrying the fixings 100.

Referring to Fig. 29, the needle 108 carrying the fixing is caused to penetrate through the pyloric sphincter tissue and into the ring 122 by advancing or sliding a second elongate support 128 distally. In the illustrated example, the needle 108 also penetrates through the ring 122, until the head 102 of the fixing passes to the opposite side. The first elongate support can remain attached to the ring 122 to provide a counter support for the ring 122 while the needle 108 is advanced.

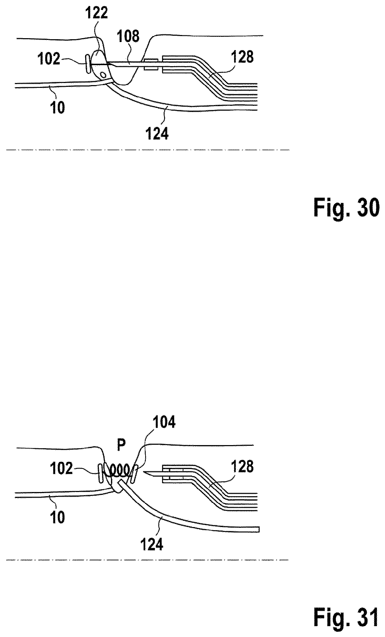

Referring to Fig. 30, the second elongate support 128 is partly retracted, allowing the head 102 of the fixing 100 to deploy laterally, and anchor against the ring 122 from the distal side.

Referring to Fig. 31, the second elongate support 128 is fully retracted, allowing the foot 104 of the fixing also to deploy laterally, and anchor against the pyloric sphincter from the opposite side to the ring 122. The security suture for the fixing may be released by pulling one end of the suture, in order to allow the needle 108 to separate from the fixing 100.

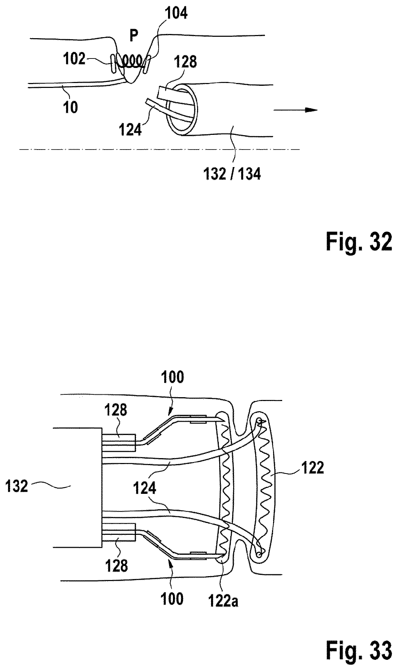

Finally in Fig. 32, the first elongate support 124 is released from the ring 122, leaving the ring 122 permanently and securely attached to the pyloric sphincter. The restraining sheath 134 can be advanced to provide an atraumatic cover over the exposed ends of the first and second elongate supports 124 and 128.

Fig. 33 illustrates a second example in which a second ring 122a is used in addition to the ring 122 to sandwich the pyloric sphincter from both sides. The second ring 122a may be carried by and/or attached to the second elongate supports 128, allowing the second ring to be guided in to contact with the pyloric sphincter when the second elongate supports 128 are advanced. Further advancement of the second elongate supports 128 causes the needles 108 to penetrate fully through the second ring 122a. For example, the second ring 122a may be attached using a suture loop similar to the loop 126 already explained for the first ring 122.

## Claims

1. A tube configured for insertion into the gastro-intestinal tract, the tube comprising an anchor (20) for anchoring the tube (10) with respect to a pylorus of a patient, the anchor comprising a gastric bulb (22) coupled or couplable to a proximal end of the tube, the gastric bulb (22) expandable from a collapsed condition for introduction into the stomach, to an operative condition within the stomach,
wherein the gastric bulb (22) comprises a fluid-tight chamber, and an inlet port (26) for (i) allowing fluid to be drawn into the chamber as the gastric bulb expands to its operative condition, and/or (ii) admitting fluid into the chamber for inflating the gastric bulb,
wherein the bulb comprises a passage configured for allowing chyme to pass through the bulb,
wherein the gastric bulb (22) is coupled to the tube by one or more tethers (18) and/or struts and **characterised in that** the tube comprises a cuff or stent (12) for resisting displacement from the implanted position towards the stomach.

2. The tube of claim 1, wherein the gastric bulb (22) is configured to partly collapse in response to stomach contractions.

3. The tube of any preceding claim, wherein the gastric bulb (22) is self-expandable from the collapsed condition to the operative condition.

4. The tube of claim 3, wherein the gastric bulb (22) comprises a self-expandable structure (24).

5. The tube of claim 4, wherein the self-expanding structure comprises a frame structure and/or a mesh structure.

6. The tube of claim 4 or 5, wherein the gastric bulb (22) has communication apertures allowing chyme to pass into the interior of the bulb.

7. The tube of claim 4-6, wherein the self-expanding structure and the fluid-tight chamber are nested one at least partly within the other.

8. The tube of claim 7, wherein the fluid-tight chamber is nested at least partly within the self-expanding structure.

9. The tube of any preceding claim, wherein the bulb has or comprises a shape that is at least one selected from: bell-shaped; tulip-shaped.

10. The tube of any preceding claim, wherein the gastric bulb (22) is directly mounted on the tube.

11. The tube according to any preceding claim, the anchor comprising a first ring (30) for fitting against a stomach-side of a pyloric sphincter, and a second ring (32) for fitting against a duodenal-side of the pyloric sphincter, the first and second rings (30, 32) coupled to one another for clamping the pyloric sphincter between the rings, the first and second rings (30, 32) having different inner and/or outer diameters from each other, optionally such that one ring has an inner diameter greater than the outer diameter of the other.

12. The tube of claim 11, wherein the first and/or second rings (30, 32) are collapsible to a collapsed condition for introduction to the pylorus.

13. The tube of claim 11 or 12, further comprising connecting elements (34) between the rings, and coupled to the rings for selectively drawing the rings closer to one another for clamping the pyloric sphincter, the connecting elements (34) optionally being filaments or sutures.

14. The tube of claim 11, 12 or 13, further comprising at least one inner ring (38) for corralling an inner peripheral edge of the pyloric sphincter, optionally wherein the at least one inner ring comprises first and second inner rings (38a, 38b).

15. The tube of claim 11, 12, 13 or 14, wherein the first and/or second ring comprises one or more of: metal; and/or a shape memory alloy optionally, nitinol; and/or plastics.

16. The tube according to any preceding claim, the anchor comprising at least one resilient element (29), expandable from a collapsed condition for introduction into the stomach, and an expanded operative condition in which the resilient element has an at least partly helix shape.

17. The tube of claim 16, wherein (i) the resilient member (29) defines a helix shape that expands in a radial direction progressively away from the proximal end of the tube; and/or (ii) the helix shape includes open space between adjacent turns or windings of the helix.

18. The tube according to any preceding claim, wherein a portion of the tube configured for fitting at the pylorus comprises reinforcement for preventing twisting of the tube.

19. The tube (10), the tube according to any preceding claim, the tube configured for insertion into the gastro-intestinal tract, the tube configured for admitting passage of stomach and/or bowel content in a first direction therethrough, and for obstructing passage of stomach and/or bowel content in an opposite second direction.

20. The tube according to any preceding claim, comprising at least one, optionally a plurality, of valving elements (56) configured for admitting passage of stomach and/or bowel content in the first direction therethrough, and for obstructing passage of stomach and/or bowel content in the second direction.

21. The tube according to claim 20, wherein the valving element (56) comprises a flap that opens to admit passage stomach and/or bowel content in the first direction through the tube,and closes to obstruct passage of stomach and/or bowel content in the second direction.

22. The tube (10) according to any preceding claim; the tube for introduction into a gastro-intestinal tract of a patient, the outer surface of the tube being provided with at least one open channel (46) extending at least partly axially and/or longitudinally with respect to the tube for permitting gastric secretions to travel outside the tube, optionally wherein the channel has a shape selected from: a helical shape around the exterior of the tube; and/or a longitudinal flute.

23. Apparatus comprising a tube (10) according to any preceding claim; the tube (10) for introduction into a gastro-intestinal tract of a patient, and the apparatus further comprising at least one gastric balloon (22) configured to bear against the stomach wall away from the pylorus, optionally for inducing a feeling of satiety in a patient.

24. Apparatus according to claim 23, wherein the balloon is filled with gas to float in the stomach and/or wherein the balloon is magnetically repulsed with respect to the tube; and/or wherein the balloon is tethered to the tube and/or wherein multiple space-occupying balloons are provided for nestling together.

## Patentansprüche

1. Röhre, die zur Einführung in den Magen-Darm-Trakt konfiguriert ist, wobei die Röhre einen Anker (20) zur Verankerung der Röhre (10) in Bezug auf einen Pylorus eines Patienten umfasst, wobei der Anker einen Magenbulbus (22) umfasst, der mit einem proximalen Ende der Röhre gekoppelt oder koppelbar ist, wobei der Magenbulbus (22) aus einem kollabierten Zustand zur Einführung in den Magen in einen Funktionszustand innerhalb des Magens expandierbar ist,
wobei der Magenbulbus (22) eine fluiddichte Kammer und eine Einlassöffnung (26) umfasst, um (i) zu ermöglichen, dass Fluid in die Kammer gesaugt wird, wenn sich der Magenbulbus in seinen Betriebszustand ausdehnt, und/oder (ii) um Fluid in die Kammer einzulassen, um den Magenbulbus zu befüllen,
wobei der Bulbus einen Durchgang aufweist, der so konfiguriert ist, dass Chymus durch den Bulbus fließen kann,
wobei der Magenbulbus (22) durch ein oder mehrere Verbindungsmittel (18) und/oder Verstrebungen mit dem Schlauch verbunden ist und **dadurch gekennzeichnet, dass**
die Röhre eine Manschette oder einen Stent (12) aufweist, um einer Verschiebung aus der implantierten Position in Richtung Magen entgegenzuwirken.

2. Schlauch nach Anspruch 1, wobei der Magenbulbus (22) so konfiguriert ist, dass er als Reaktion auf Magenkontraktionen teilweise kollabiert.

3. Die Röhre nach einem der vorhergehenden Ansprüche, wobei der Magenbulbus (22) vom kollabierten Zustand bis zum Betriebszustand selbstexpandierbar ist.

4. Röhre nach Anspruch 3, wobei der Magenbulbus (22) eine selbstexpandierbare Struktur (24) aufweist.

5. Die Röhre nach Anspruch 4, wobei die selbstexpandierende Struktur eine Rahmenstruktur und/oder eine Netzstruktur umfasst.

6. Röhre nach Anspruch 4 oder 5, wobei der Magenbulbus (22) Verbindungsöffnungen aufweist, durch die Speisebrei in das Innere des Bulbus gelangen kann.

7. Röhre nach Anspruch 4-6, wobei die selbstexpandierende Struktur und die fluiddichte Kammer zumindest teilweise ineinander verschachtelt sind.

8. Röhre nach Anspruch 7, wobei die fluiddichte Kammer zumindest teilweise in der selbstexpandierenden Struktur untergebracht ist.

9. Die Röhre nach einem der vorhergehenden Ansprüche, wobei der Bulbus mindestens eine der folgenden Formen hat oder aufweist: glockenförmig; tulpenförmig.

10. Die Röhre nach einem der vorhergehenden Ansprüche, wobei der Magenbulbus (22) direkt an der Röhre angebracht ist.

11. Röhre nach einem der vorhergehenden Ansprüche, wobei der Anker einen ersten Ring (30) zum Anlegen an eine Magenseite eines Pylorus-Schließmuskels und einen zweiten Ring (32) zum Anlegen an eine Duodenalseite des Pylorus-Schließmuskels umfasst, wobei der erste und der zweite Ring (30, Der erste und der zweite Ring (30, 32) sind miteinander gekoppelt, um den Pylorus-Schließmuskel zwischen den Ringen einzuklemmen, wobei der erste und der zweite Ring (30, 32) unterschiedliche Innen- und/oder Außendurchmesser haben, optional so, dass ein Ring einen größeren Innendurchmesser als der Außendurchmesser des anderen hat.

12. Röhrchen nach Anspruch 11, wobei der erste und/oder der zweite Ring (30, 32) zum Einführen in den Pylorus in einen zusammengefalteten Zustand gebracht werden können.

13. Röhre nach Anspruch 11 oder 12, ferner mit Verbindungselementen (34) zwischen den Ringen, die mit den Ringen gekoppelt sind, um die Ringe zum Festklemmen des Pylorusschließmuskels selektiv näher zueinander zu ziehen, wobei die Verbindungselemente (34) wahlweise Fäden oder Nähte sind.

14. Röhre nach Anspruch 11, 12 oder 13, die ferner mindestens einen Innenring (38) zum Umschließen einer inneren Umrandung des Pylorussphinkters umfasst, wobei der mindestens eine Innenring optional einen ersten und einen zweiten Innenring (38a, 38b) umfasst.

15. Röhre nach Anspruch 11, 12, 13 oder 14, wobei der erste und/oder zweite Ring eines oder mehrere der folgenden Elemente umfasst: Metall und/oder eine Formgedächtnislegierung, optional Nitinol, und/oder Kunststoff.

16. Röhre nach einem der vorhergehenden Ansprüche, wobei der Anker mindestens ein flexibles Element (29) umfasst, das aus einem kollabierten Zustand zum Einführen in den Magen und einem expandierten Betriebszustand, in dem das flexible Element eine zumindest teilweise Helixform aufweist, expandierbar ist.

17. Röhre nach Anspruch 16, wobei (i) das flexible Element (29) eine Helixform definiert, die sich in einer radialen Richtung progressiv vom proximalen Ende der Röhre weg ausdehnt; und/oder (ii) die Helixform einen offenen Raum zwischen benachbarten Windungen der Helix einschließt.

18. Die Röhre nach einem der vorhergehenden Ansprüche, wobei ein Abschnitt der Röhre, der für die Anpassung an den Pylorus konfiguriert ist, eine Verstärkung aufweist, um ein Verdrehen der Röhre zu verhindern.

19. Röhre (10) nach einem der vorhergehenden Ansprüche, wobei die Röhre zum Einführen in den Magen-Darm-Trakt konfiguriert ist, wobei die Röhre so konfiguriert ist, dass sie den Durchgang von Magen- und/oder Darminhalt in einer ersten Richtung durch sie hindurch zulässt und den Durchgang von Magen- und/oder Darminhalt in einer entgegengesetzten zweiten Richtung blockiert.

20. Röhre nach einem der vorhergehenden Ansprüche, umfassend mindestens ein, optional eine Vielzahl von Ventilelementen (56), die so konfiguriert sind, dass sie den Durchgang von Magen- und/oder Darminhalt in der ersten Richtung ermöglichen und den Durchgang von Magen- und/oder Darminhalt in der zweiten Richtung blockieren.

21. Röhre nach Anspruch 20, wobei das Ventilelement (56) eine Klappe umfasst, die sich öffnet, um den Durchgang von Magen- und/oder Darminhalt in der ersten Richtung durch die Röhre zu ermöglichen, und sich schließt, um den Durchgang von Magen- und/oder Darminhalt in der zweiten Richtung zu blockieren.

22. Röhre (10) nach einem der vorhergehenden Ansprüche, wobei die Röhre zum Einführen in den Magen-Darm-Trakt eines Patienten bestimmt ist und die Außenfläche der Röhre mit mindestens einem offenen Kanal (46) versehen ist, der sich zumindest teilweise axial und/oder in Längsrichtung der Röhre erstreckt, damit Magensekrete aus der Röhre austreten können, und wobei der Kanal gegebenenfalls eine Form hat, die ausgewählt ist aus: einer spiralförmigen Form um die Außenseite der Röhre herum und/oder einer Längsrinne.

23. Vorrichtung mit einer Röhre (10) nach einem der vorhergehenden Ansprüche, wobei die Röhre (10) zum Einführen in den Magen-Darm-Trakt eines Patienten bestimmt ist und die Vorrichtung ferner mindestens einen Magenballon (22) umfasst, der so konfiguriert ist, dass er an der vom Pylorus entfernten Magenwand anliegt, um optional ein Sättigungsgefühl bei einem Patienten hervorzurufen.

24. Vorrichtung nach Anspruch 23, bei der der Ballon mit Gas gefüllt ist, um im Magen zu schwimmen, und/oder bei der der Ballon in Bezug auf die Röhre magnetisch abgestoßen wird, und/oder bei der der Ballon an der Röhre angebunden ist, und/oder bei der mehrere raumfüllende Ballons zum Zusammenfügen vorgesehen sind.

## Revendications

1. Tube configuré pour être inséré dans le tractus gastro-intestinal, le tube comprenant une ancre (20) pour ancrer le tube (10) par rapport au pylore d'un patient, l'ancre comprenant une ampoule gastrique (22) couplée ou couplable à une extrémité proximale du tube, l'ampoule gastrique (22) extensible d'un état replié pour l'introduction dans l'estomac, à un état opérationnel à l'intérieur de l'estomac,
dans lequel l'ampoule gastrique (22) comprend une chambre étanche et un orifice d'entrée (26) permettant (i) d'aspirer du liquide dans la chambre lorsque l'ampoule gastrique se dilate jusqu'à sa condition opérationnelle, et/ou (ii) d'admettre du fluide dans la chambre pour faire gonfler l'ampoule gastrique, l'ampoule comprend un passage configuré pour permettre au chyme de passer à travers l'ampoule,
dans lequel l'ampoule gastrique (22) est couplée au tube par une ou plusieurs attaches (18) et/ou entretoises et **caractérisé en ce que**
le tube comprend une cuff ou une stent (12) pour résister au déplacement de la position implantée vers l'estomac.

2. Tube de la revendication 1, dans lequel l'ampoule gastrique (22) est configurée pour se réduire partiellement en réponse aux contractions de l'estomac.

3. Tube de toute revendication précédente, dans lequel l'ampoule gastrique (22) est auto-expansible de l'état réduit à l'état opérationnel.

4. Le tube de la revendication 3, dans lequel l'ampoule gastrique (22) comprend une structure auto-expansible (24).

5. Le tube de la revendication 4, dans lequel la structure auto-expansive comprend une structure de cadre et/ou une structure de mesh.

6. Tube de la revendication 4 ou 5, dans lequel l'ampoule gastrique (22) présente des ouvertures de communication permettant le passage du chyme à l'intérieur de l'ampoule.

7. Le tube des revendications 4 à 6, dans lequel la structure auto-expansive et la chambre étanche au fluide sont emboîtées l'une au moins partiellement dans l'autre.

8. Le tube de la revendication 7, dans lequel la chambre étanche aux fluides est emboîtée au moins en partie dans la structure auto-expansive.

9. Le tube de toute revendication précédente, dans lequel l'ampoule a ou comprend une forme qui est au moins choisie parmi : en forme de cloche ; en forme de tulipe.

10. Le tube de toute revendication précédente, dans lequel l'ampoule gastrique (22) est montée directement sur le tube.

11. Tube selon toute revendication précédente, l'ancre comprenant un premier anneau (30) pour s'adapter à un côté stomacal du sphincter pylorique, et un second anneau (32) pour s'adapter à un côté duodénal du sphincter pylorique, le premier et le second anneaux (30, 32) couplés l'un à l'autre pour serrer le sphincter pylorique entre les anneaux, le premier et le second anneau (30, 32) ayant des diamètres intérieurs et/ou extérieurs différents l'un de l'autre, éventuellement de telle sorte qu'un anneau ait un diamètre intérieur supérieur au diamètre extérieur de l'autre.

12. Le tube de la revendication 11, dans lequel le premier anneau et/ou le second anneau (30, 32) sont repliables à un état réduit pour l'introduction dans le pylore.

13. Le tube de la revendication 11 ou 12, comprenant en outre des éléments de connexion (34) entre les anneaux, et couplés aux anneaux pour rapprocher sélectivement les anneaux l'un de l'autre afin de serrer le sphincter pylorique, les éléments de connexion (34) pouvant être des filaments ou des sutures.

14. Le tube de la revendication 11, 12 ou 13, comprenant en outre au moins un anneau intérieur (38) pour encercler un bord périphérique intérieur du sphincter pylorique, éventuellement dans lequel l'au moins un anneau intérieur comprend un premier et un second anneau intérieur (38a, 38b).

15. Le tube de la revendication 11, 12, 13 ou 14, dans lequel le premier anneau et/ou le deuxième anneau comprend un ou plusieurs des éléments suivants : métal ; et/ou un alliage à mémoire de forme éventuellement, nitinol ; et/ou des matières plastiques.

16. Tube selon toute revendication précédente, l'ancre comprenant au moins un élément résilient (29), extensible à partir d'un état réduit pour l'introduction dans l'estomac, et un état opérationnel expansé dans lequel l'élément résilient a une forme d'hélice au moins en partie.

17. Le tube de la revendication 16, dans lequel (i) l'élément résilient (29) définit une forme d'hélice qui s'étend dans une direction radiale s'éloignant progressivement de l'extrémité proximale du tube ; et/ou (ii) la forme d'hélice comprend un espace ouvert entre les tours adjacents ou les enroulements de l'hélice.

18. Le tube selon toute revendication précédente, dans lequel une partie du tube configurée pour s'adapter au pylore comprend un renforcement pour empêcher la torsion du tube.

19. Le tube (10), le tube selon toute revendication précédente, le tube configuré pour être inséré dans le tractus gastro-intestinal, le tube configuré pour admettre le passage du contenu de l'estomac et/ou de l'intestin dans une première direction à travers lui, et pour obstruer le passage du contenu de l'estomac et/ou de l'intestin dans une seconde direction opposée.

20. Le tube selon toute revendication précédente, comprenant au moins un, optionnellement plusieurs, éléments de valve (56) configurés pour admettre le passage du contenu de l'estomac et/ou de l'intestin dans la première direction à travers lui, et pour obstruer le passage du contenu de l'estomac et/ou de l'intestin dans la seconde direction.

21. Le tube selon la revendication 20, dans laquelle l'élément de valve (56) comprend un clapet qui s'ouvre pour permettre le passage de l'estomac et/ou du contenu intestinal dans la première direction à travers la sonde, et qui se ferme pour obstruer le passage de l'estomac et/ou du contenu intestinal dans la seconde direction.

22. Tube (10) selon l'une quelconque des revendications précédentes, le tube étant destiné à être introduit dans le tractus gastro-intestinal d'un patient, la surface extérieure du tube étant pourvue d'au moins un canal ouvert (46) s'étendant au moins en partie axialement et/ou longitudinalement par rapport au tube pour permettre aux sécrétions gastriques de s'écouler à l'extérieur du tube, le canal ayant éventuellement une forme choisie parmi : une forme hélicoïdale autour de l'extérieur du tube ; et/ou une cannelure longitudinale.

23. Appareil comprenant un tube (10) selon toute revendication précédente ; le tube (10) destiné à être introduit dans le tractus gastro-intestinal d'un patient, et l'appareil comprenant en outre au moins un ballon gastrique (22) configuré pour s'appuyer contre la paroi de l'estomac à l'écart du pylore, éventuellement pour induire une sensation de satiété chez un patient.

24. Appareil selon la revendication 23, dans lequel le ballon est rempli de gaz pour flotter dans l'estomac et/ou dans lequel le ballon est repoussé magnétiquement par rapport au tube ; et/ou dans lequel le ballon est attaché au tube et/ou dans lequel plusieurs ballons occupant de l'espace sont prévus pour s'emboîter les uns dans les autres.
